(19) Europäisches Patentamt
European Patent Office
Office européen des brevets

(11) **EP 1 990 027 B1**

(12) **EUROPÄISCHE PATENTSCHRIFT**

(45) Veröffentlichungstag und Bekanntmachung des Hinweises auf die Patenterteilung:
**28.10.2015 Patentblatt 2015/44**

(51) Int Cl.:
*A61F 2/86* (2013.01)     *A61B 5/055* (2006.01)
*A61B 5/07* (2006.01)     *G01R 33/28* (2006.01)

(21) Anmeldenummer: **08075256.1**

(22) Anmeldetag: **31.03.2008**

(54) **Medizinisches Implantat, insbesondere Stent zum Einsatz in Körperlumen**

Medical implant, in particular stent for use in body lumen

Implant médical, en particulier stent destiné à l'utilisation dans des lumières corporelles

(84) Benannte Vertragsstaaten:
**CH DE FR GB IE LI**

(30) Priorität: **09.05.2007 DE 102007021692**

(43) Veröffentlichungstag der Anmeldung:
**12.11.2008 Patentblatt 2008/46**

(73) Patentinhaber: **Biotronik VI Patent AG**
**6341 Baar (CH)**

(72) Erfinder:
• **Philipp, Jens, Dr.**
**10555 Berlin (DE)**

• **Neumann, Andreas**
**10969 Berlin (DE)**

(74) Vertreter: **Galander, Marcus et al**
**Biotronik SE & Co. KG**
**Corporate Intellectual Properties**
**Woermannkehre 1**
**12359 Berlin (DE)**

(56) Entgegenhaltungen:
**WO-A1-01/12092     WO-A1-2006/083478**
**WO-A2-2007/109756  DE-A1- 19 746 735**
**US-A1- 2004 082 867  US-A1- 2005 159 802**

**Beschreibung**

Die Erfindung betrifft ein medizinisches Implantat und insbesondere einen Stent zum Einsatz in Körperlumen.

Derartige Implantate werden beispielsweise in Form des erwähnten Stents zur Therapie von Stenosen in Koronargefäße mit Hilfe eines Katheters eingesetzt. Andere Anwendungsgebiete derartiger Implantate sind beispielsweise so genannte Aneurysma-Coils.

Bei derartigen Implantaten besteht nach der Implantation grundsätzlich das Problem, dass der Zustand des Implantates insbesondere im zeitlichen Verhalten nach dem Einsetzen überwachbar sein soll. Hierzu ist es bekannt, bildgebende Verfahren einzusetzen, wie beispielsweise eine Röntgenuntersuchung, bei der zur besseren Darstellung des Implantates oftmals Kontrastmittel gespritzt werden müssen. Sowohl die Röntgenstrahlenbelastung als auch die Applikation eines Kontrastmittels stellen für den Körper des Patienten eine nicht wünschenswerte Belastung dar. Ferner ist der Zustand von Implantaten, die insbesondere aus metallischen Legierungen mit überwiegendem Anteil aus Elementen niedriger Ordnungszahl bestehen (wie z.B. Mg), angiographisch grundsätzlich kaum detektierbar.

Im Zusammenhang mit der Zustandsüberwachung des Implantates sind verschiedenartige Zielsetzungen zu nennen. So ist die Feststellung von Schäden, wie ein Abriss von Streben der Basisstruktur des Implantates oder die Erkennung von Strebenrissen insbesondere bei nicht degradierbaren Implantaten von Interesse.

Bei degradierbaren Implantaten ist der aktuelle Degradationszustand des Implantates und der zeitliche Verlauf der Degradation eine wichtige Information.

Schließlich sind noch Implantate mit Medikamentendepots zu nennen, bei denen ein wichtiger Fragenkomplex umfasst, ob das deponierte Medikament bereits abgegeben wird und in welchem Maße dies bereits erfolgt ist.

Die oben erwähnten Untersuchungsmethoden auf der Basis von Röntgenmarkern und Kontrastmitteln können nur wenige Informationen geben und sind - wie erwähnt - belastend für den Patienten.

Zur Lösung der vorstehenden Problematik wird in der Literatur die grundsätzliche Möglichkeit diskutiert, aktive Sensoren auf dem Implantat, wie beispielsweise einen Stent, zu applizieren. Dies ist jedoch gerade bei bio-degradierbaren Stents nicht möglich, da derartige Sensoren auf Halbleitermaterialien basieren, die nicht abbaubar sind und in der Regel auch giftige Materialien enthalten, so etwa durch die Dotierung des Halbleitermaterials.

Ausgehend von der geschilderten Problematik liegt der Erfindung die Aufgabe zugrunde, ein medizinisches Implantat so auszurüsten, dass sein Zustand unter Einsatz vergleichsweise einfacher technischer Mittel ohne die erwähnten belastenden Untersuchungsverfahren erfassbar ist.

Diese Aufgabe wird durch die im Kennzeichnungsteil des Anspruches 1 angegebenen Merkmale gelöst, wonach in die Basisstruktur eine passive elektronische Schwingkreis-Anordnung aus mindestens einer Induktivität und mindestens einem Kondensator integriert ist. Die Eigenfrequenz dieser Schwingkreis-Anordnung im implantierten Zustand des Implantats ist von außen durch eine Anregung mittels einer physiologisch unbedenklichen elektromagnetischen Strahlung erfassbar, so dass ein signifikantes Identifikationsmerkmal oder eine Veränderung der Basisstruktur durch den Wert oder eine Änderung der Eigenfrequenz der Schwingkreis-Anordnung detektierbar sind.

Das Wesen der Erfindung liegt also darin, dass das Implantat selbst zu einem passiven elektromagnetischen Schwingkreis quasi "umfunktioniert" wird, der durch eine medizinisch unkritische elektromagnetische Strahlung "abfragbar" ist. Es handelt sich bei der erfindungsgemäßen Konstruktion also nicht um eine Hybridlösung, bei er ein Schwingkreis aus eigens hierfür bestimmten Bauteilen am Stent angebracht wird. Vielmehr bildet der Stent selbst den Schwingkreis. Dieser ist somit - bildlich gesprochen - als "Stent mit passiver Intelligenz" zu bezeichnen.

Die Basisstruktur von Stents kann aus tragenden und nicht-tragenden Elementen bestehen, wobei die Erfindung in beiden Typen dieser Elemente implementiert werden kann.

Gemäß einer beispielhaften Ausgestaltung kann die Eigenfrequenz der Schwingkreis-Anordnung signifikant für den Typ und gar die Seriennummer des Implantats sein. Dies wird beispielsweise dadurch realisiert, dass ein Implantat eines bestimmten Typs mit einer Schwingkreis-Anordnung einer dafür repräsentativen Eigenfrequenz aufgebaut wird, die nach der Implantation von außen abfragbar ist.

Gemäß einer weiteren bevorzugten Ausführungsform lässt sich aus einer Änderung der Eigenfrequenz der Schwingkreis-Anordnung nach der Implantation des Implantats auf eine Beschädigung der Basisstruktur schließen. Dies erfolgt beispielsweise dadurch, dass bei Abreißen einer einen Kondensator bildenden Strebe der Basisstruktur der Schwingkreis unterbrochen wird und dadurch eine Eigenfrequenz nicht mehr messbar ist.

Anwendungstechnisch besonders interessant ist die erfinfungsgemässe Ausführungsform, bei der eine Änderung der Eigenfrequenz der Schwingkreis-Anordnung nach der Implantation des Implantats signifikant ist für das Maß der Biodegradierung der Basisstruktur. Hierbei werden ein, vorzugsweise mehrere Kondensatoren zusammen mit mindestens einer Induktivität zu einem Schwingkreis verschaltet, wobei die Hülle des Implantats durch eine die Schwingkreis-Anordnung abdeckende, bio-degradierbare Isolation gebildet ist. Mit dem fortschreitenden Zerfall dieser Isolation werden die Kondensatoren nach und nach gegenüber der im Körperlumen fließenden, physiologischen, elektrisch leitfähigen Flüssigkeit freigelegt, so dass der entsprechende Kondensator kurzgeschlossen wird. Jeder Kurzschluss eines Kondensators verändert dann die Gesamtkapazität der in Reihe geschalteten Kondensatoren und damit die Eigen- oder Resonanzfrequenz des Schwingkreises. Die Eigenfrequenz wird also bestimmt durch die noch intakten, isolierten Kondensatoren. Der Kurzschluss eines Kondensators wirkt also praktisch wie ein Schalter, der den Kondensator aus der Reihenschaltung herausnimmt, wodurch die Eigenfrequenz sprunghaft geändert wird.

Um in diesem Zusammenhang das Degradationsverhalten möglichst genau nachvollziehen zu können, ist es von Vorteil, wenn jeder Kondensator zu einem unterschiedlichen und ungefähr definierten Zeitpunkt nach der Implantation "schaltet", also kurzgeschlossen wird. Dies kann bei der Herstellung beispielsweise durch eine Variation der Isolationsdicke an jeden Kondensator eingestellt werden. Alternativ dazu ist es auch möglich, die Dichte des Isolationsmaterials oder die Materialart selbst zu variieren. Durch eine geeignete Dimensionierung der Kondensatoren kann bei Bedarf eindeutig festgestellt werden, welcher Kondensator bereits kurzgeschlossen ist. Kommt es nur darauf an zu wissen, wie viele Kondensatoren kurzgeschlossen sind, so reicht eine gleiche Dimensionierung für alle Kondensatoren aus, wie dies im Ausführungsbeispiel unten noch näher erläutert wird.

Gemäß einer weiteren bevorzugten Ausführungsform der Erfindung kann eine Änderung der Eigenfrequenz der Schwingkreis-Anordnung nach der Implantation des Implantats signifikant für den Status eines in das Implantat integrierten, sich zeitlich verzögert abbauenden Medikamentendepots sein. Dazu ist - wie im Ausführungsbeispiel noch näher erläutert wird - dem Medikamentendepot wiederum mindestens ein Kondensator in geeigneter Weise zuzuordnen, der aufgrund des Abbaus des Medikamentendepots synchron freigelegt wird und damit entsprechend seine Kapazität ändert, was sich wiederum auf die Eigenfrequenz des Schwingkreises in von außen detektierbarer Weise auswirkt.

Bevorzugterweise wird die Induktivität der Schwingkreis-Anordnung durch eine elektrisch leitfähige Strebe der Basisstruktur gebildet. Dies ist auf einfache Weise realisierbar, da derartige Streben in der Regel in Peripherrichtung umlaufen und somit bereits auf ihrer grundlegenden Formgebung beruhend eine Spulenwindung bilden.

Der mindestens eine Kondensator der Schwingkreis-Anordnung wird vorzugsweise durch einen Lagenaufbau einer Strebe der Basisstruktur realisiert. Die Strebe besteht demnach aus einer inneren Isolationslage, einer ersten elektrisch leitfähigen Lage als ersten Kondensatorpol, einer vorzugsweise als Dielektrikum ausgebildeten Zwischenlage, einer zweiten elektrisch leitfähigen Lage als zweiten Kondensatorpol und einer äußeren, ggf. aus bio-degradierbarem Material bestehenden Isolationslage. Im Ausführungsbeispiel ist dies noch näher beschrieben.

Zur Abfrage des Schwingkreises kann ein externes Abfragegerät eingesetzt werden, das eine variable elektromagnetische Strahlung zur Anregung des Schwingkreises aussendet. Geeignete Frequenzbereiche zum Senden von elektromagnetischen Signalen durch den menschlichen Körper liegen im Bereich von einigen Kilohertz bis hin zu ca. 5 Gigahertz, wobei die zu benutzenden Strukturgrössen des Implantates einen schmaleren Bereich vorgeben werden. Im unten stehenden Ausführungsbeispiel liegt der Resonanzbereich zwischen ca. 300 MHz und ca.700 Mhz.

In dem Abfragegerät kann beispielsweise ein Frequenz-Sweep-Generator zum Einsatz kommen, der das gesamte mögliche Frequenzband, in dem die Eigenfrequenzen der Schwingkreis-Anordnung je nach Zustand der daran beteiligten passiven Komponenten liegen, überstreicht. Das Abfragegerät detektiert dann die Resonanzantwort der Schwingkreis-Anordnung. Das Abfragegerät kann so anhand des Wertes der Resonanzantwort des Implantats daraus eine entsprechende signifikante Information gewinnen. Bei mehreren, im Körper nahe zusammen liegenden Stents mit "passiver Intelligenz" ist es dabei von Vorteil, dass die Richtcharakteristik des im Abfragegerät arbeitenden Senders möglich gebündelt ist.

Alternativ zu einem externen Abfragegerät kann auch ein aktives medizinisches Implantat, wie ein Herzschrittmacher, Defibrillator, Medikamentenpumpe, Neurostimulator usw. mit einem entsprechenden Sender-Empfänger-Gerät ausgerüstet sein. Dies ist insbesondere von Vorteil für bekannte Implantate mit telemedizinischer Datenübertragung. Diese sogenannten "Tranceiver" können so modifiziert werden, dass sie den gewünschten Frequenzbereich der Schwingkreis-Anordnung überstreichen können.

Zusammenfassend kann im Hinblick auf das quasi digitale Schaltverhalten des mindestens einen Kondensators durch dessen Kurzschluss bei Freilegung gegenüber der leitfähigen physiologischen Flüssigkeit im Bereich des Stents eine gut reproduzierbare Abfragecharakteristik erzielt werden. Jeder Kurzschluss eines Kondensators erzeugt einen signifikanten Sprung der Resonanzfrequenz, wobei die Anordnung sehr tolerant gegenüber Fertigungsungenauigkeiten ist. Schwankende Werte der beteiligten Komponenten des Schwingkreises ändern zwar die Resonanzfrequenzen, so lang diese Abweichungen jedoch in ihrem Maß deutlich unterhalb der Frequenzverschiebung zwischen zwei Resonanzfrequenzen bleiben, stellt dies kein die Funktionsfähigkeit der Anordnung bedrohendes Problem dar.

Weitere Merkmale, Einzelheiten und Vorteile der Erfindung ergeben sich aus der nachfolgenden Beschreibung, in der ein Ausführungsbeispiel anhand der beigefügten Zeichnungen näher erläutert wird. Es zeigen:

Fig. 1a       eine ausschnittsweise Ansicht eines Stents mit "passiver Intelligenz",

Fig. 1b       eine schematische perspektivische Darstellung einer Schwingkreis-Anordnung,

Fig. 2a bis 2e       Ersatzschaltbilder einer in den Stent integrierten Schwingkreis-Anordnung in verschiedenen Kurzschlusssituationen,

Fig. 2f       ein Frequenzdiagramm der Resonanzantworten des Schwingkreises in den unterschiedlichen Kurzschlusssituationen gemäß Fig. 2a bis 2e,

Fig. 3a bis 3d     schematische Querschnitte durch eine Strebe des Stents gemäß Fig. 1,

Fig. 4     eine schematische Ansicht eines Patienten mit implantiertem Stent und Abfragegerät,

Fig. 5a bis 5c     Darstellungen von Anzeigen am Abfragegerät in Abhängigkeit unterschiedlicher implantierter Stents,

Fig. 6     eine schematische Ansicht eines Patienten mit implantierten Stent und aktivem medizinischen Implantat,

Fig. 7a bis 7f     schematische Diagramme zur Darlegung eines Herstellungsverfahrens für einen Stent mit Schwingkreis-Anordnung,

Fig. 8     einen schematischen Querschnitt durch den Stent,

Fig. 9     eine schematische Ansicht zweier Stentstreben mit angedeuteten Kondensatoren der Schwingkreis-Anordnung,

Fig. 10a bis 10c     Querschnitte durch Streben eines Stents mit einem Medikamentendepot und zugeordneten Schwingkreis-Anordnungen.

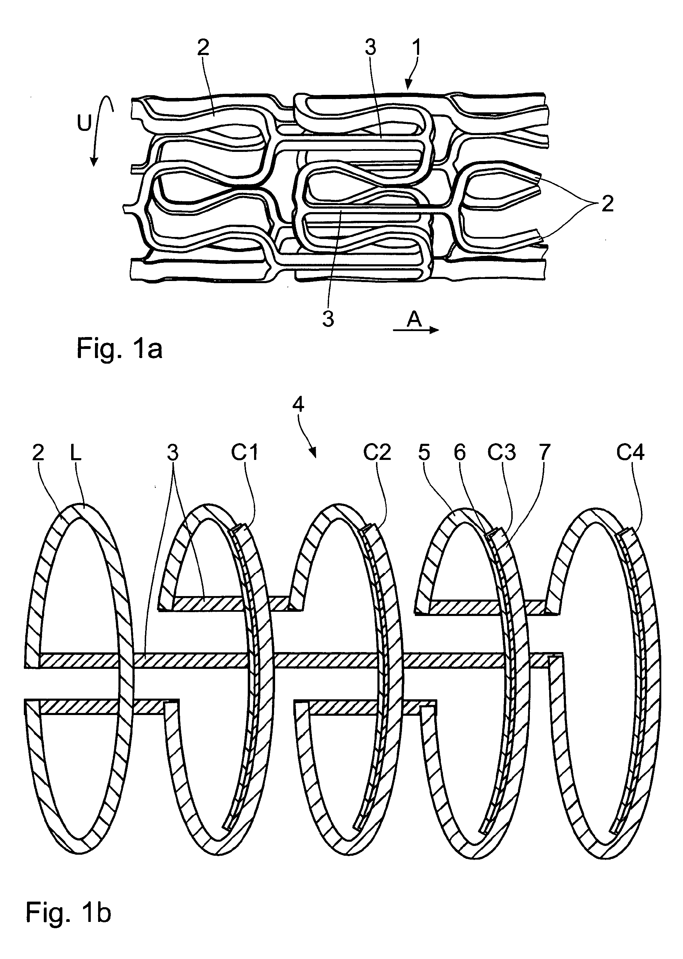

Der in Fig. 1 ausschnittsweise gezeigte Stent 1 besteht hinsichtlich seiner Basisstruktur aus in Umfangrichtung umlaufenden, mäanderförmigen Streben 2 und diese verbindende Axialverbinder 3. Bei der Erfindung kommt es in diesem Zusammenhang nicht auf die spezielle Formgebung der Streben 2 und Axialverbinder 3 an, vielmehr ist die Erfindung innerhalb des Aufbaus der Streben 2 oder Axialverbinder 3 realisiert, wie im folgenden noch deutlich wird.

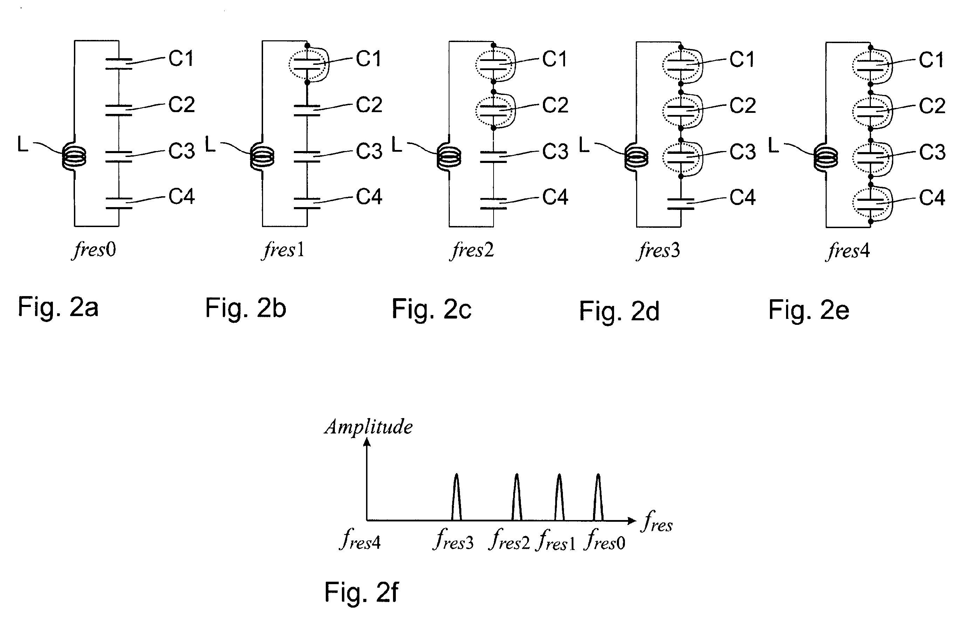

Wie in Fig. 2a angedeutet ist, ist in den Stent 1 eine passive elektronische Schwingkreis-Anordnung 4 durch einen noch näher zu erläuternden Aufbau der Streben 2 bzw. Axialverbinder 3 integriert, die aus einer Induktivität L und Kondensatoren C1 bis C4 in Reihenschaltung besteht. Wie aus Fig. 1b schematisch hervorgeht, sind die Kondensatoren C1 bis C4 und die Induktivität L durch eine entsprechende Ausbildung der als Ganzes mit 2 bezeichneten Streben realisierbar. Grundsätzlich bestehen die einzelnen Kondensatoren C1 bis C4 aus einer teilringförmigen inneren elektrisch leitfähigen Lage 5, einer isolierenden, als Dielektrikum ausgebildeten Zwischenlage 6 und einer zweiten elektrisch leitfähigen, teilringförmigen Lage 7, die mit der Zwischenlage 6 und der inneren Lage 5 zumindest teilweise überlappt. Über die Axialverbinder 3 sind diese einzelnen Kondensatoranordnungen C1 bis C4 in Axialrichtung A miteinander elektrisch in Reihe geschaltet. Die Schwingkreis-Anordnung 4 wird durch die Induktivität L komplettiert, die aus einer um den Stent in Umfangsrichtung U herumgeführten, elektrisch leitfähigen Strebe 2 besteht.

Diese grundsätzliche Schwingkreis-Anordnung ist im Ausgangszustand in eine isolierende, bio-degradierbare Hülle eingeschlossen, durch deren Abbau die einzelnen Kondensatoren C1 bis C4 in signifikanter Weise freigelegt und damit durch beispielsweise das den Stent umfließende Blut kurzgeschlossen werden. Dies ist in den Fig. 2a bis 2e angedeutet.

Im Folgenden wird die Auslegung der Kondensatoren C1 bis C4 und der Einfluss deren Kurzschlusses auf die Eigenfrequenz der Schwingkreis-Anordnung 4 näher erläutert werden.

Hier soll beispielhaft ein koronarer Stent mit Anzeige seines Degradationszustandes in 25% Schritten dimensioniert werden. Die Anordnung der Bauteile ist in Fig. 1b schematisch dargestellt.

Nur quantitativ betrachtet wird hier der Einfluss parasitärer Grössen, wie elektrische Widerstände Rpar, Kapazitäten Cpar und Induktivitäten Lpar. Diese können Auswirkungen auf die Resonanzfrequenz fres, sowie die Schwingkreisgüte Q haben. Solche parasitären Parameter entstehen beispielsweise durch den Einfluss der Körperflüssigkeit und des Körpergewebes, die den Stent umgeben. Diese weisen eine elektrische Impedanz Z sich ergebend aus Rpar, Cpar, Lpar auf, die mit dem Schwingkreis gekoppelt ist. Dadurch werden die Eigenschaften des Schwingkreises wie die Resonanzfrequenz fres und die Güte Q beeinflusst. Der Einfluss dieser parasitären Größen muss beim Entwurf eines solchen Implantates berücksichtigt werden.

Die nachfolgende Rechnung ist also idealisiert.

Angenommen wird eine Luftspule, die einmal um den Stent-Umfang umläuft. Die sich daraus ergebende Induktivität ergibt sich wie folgt:

$$L = \mu \cdot \mu_r \frac{N^2 \cdot A}{h} \left[ H \right]$$

$$\mu = 4 \cdot \Pi \cdot 10^{-7} \left[ \frac{Vs}{Am} \right]$$

$$\mu_r = 1 (Wasser)$$

$$A = Grundfläche\ des\ Stentquerschnitts$$

$$N = Anzahl\ der\ Windungen$$

$$r = 1mm = 10^{-3}m\,(Coronarstent)$$

$$h = 0{,}1mm = 10^{-4}m$$

$$A = \Pi r^2$$

$$A = \Pi \cdot 10^{-6}\left[m^2\right]$$

$$L = 4\Pi \cdot 10^{-7}\frac{\Pi \cdot 10^{-6}}{10^{-4}}[H]$$

$$L = 4\Pi^2 \cdot 10^{-9}[H]$$

$$L = 40 \cdot 10^{-9}[H]$$

$$\underline{\underline{L = 40[nH]}}$$

Um die Konstruktion des Stents und das Rechenbeispiel einfach zu halten, wird hier von vier gleichen Kondensatoren ausgegangen. Das bedeutet, dass von außen feststellbar ist, wie viele Schalter schon geschlossen sind, aber nicht welche. Wenn das auch interessiert, müssen die Kondensator unterschiedliche Kapazitäten haben. Dies kann z.B. durch Variation der Dimensionen der Stege geschehen.

Um beispielhaft eine mögliche Kapazität auf einem Steg eines Koronar-Stents zu ermitteln wird von nachfolgenden Strukturgrößen ausgegangen:

$$b = 100[\mu m] = 10^{-4}\,[m]\ \ \text{Breite eines Stentstegs}$$

$$l = 5[mm] = 5 \cdot 10^{-3}\,[m]\ \ \text{Länge eines Stentstegs}$$

$$d = 10[\mu m] = 10^{-5}\,[m]\ \ \text{Breite des Dielektrikums}$$

$$\varepsilon_0 = 1{,}112 \cdot 10^{-10}\,[\frac{F}{m}]\ \ \text{Dielektrizitätskonstante}$$

$$C = \frac{\varepsilon_0 \cdot \varepsilon_r \cdot b \cdot l}{d}$$

$$C = \frac{1{,}112 \cdot 10^{-10} \cdot 5 \cdot 10^{-3} \cdot 10^{-3}}{10^{-4}} [F]$$

$$C = 5{,}6 \cdot 10^{-12} [F]$$

$$\underline{\underline{C = 5{,}6 [pF]}} \; \text{pro Steg - Kondensator}$$

Die Eigenfrequenz $f_{res}$ des Stents ergibt sich wie folgt:

Allgemein gilt für die Resonanzfrequenz

$$f_{res} = \frac{1}{2\Pi \cdot \sqrt{L \cdot C}} [Hz]$$

Bei der Reihenschaltung von Kondensatoren gilt :

$$C_{ges} = \left( \frac{1}{C_1} + \frac{1}{C_2} + \frac{1}{C_3} + ... + \frac{1}{Cn} \right)^{-1}$$

Da in diesem Rechenbeispiel alle Kondensatoren den gleichen Wert haben sollen, gilt

$$C_{ges} = \frac{1}{n} C$$

Mit den 40 nH für die Spule vereinfacht sich die Formel zu

$$f_{resn} = \frac{800}{\sqrt{\frac{1}{n} C}} [Hz]$$

Die Eigenfrequenzen $f_{res4}$, $f_{res3}$, $f_{res2}$, $f_{res1}$, $f_{res0}$ für jeden der fünf möglichen Zustände berechnen sich wie folgt: Eingesetzt in die Resonanzfrequenformel ergibt sich :

Für vier intakte Kondensatoren :

$$f_{res4} = \frac{800}{\sqrt{\frac{1}{4} C}} [Hz] = 673 MHz$$

Für drei intakte Kondensatoren :

$$f_{res3} = \frac{800}{\sqrt{\frac{1}{3} C}} [Hz] = 581 MHz$$

Für zwei intakte Kondensatoren :

$$f_{res2} = \frac{800}{\sqrt{\frac{1}{2} C}} [Hz] = 475 MHz$$

(fortgesetzt)

Für einen intakten Kondensator :

$$f_{res1} = \frac{800}{\sqrt{\frac{1}{1}C}}[Hz] = 336MHz$$

Für keinen intakten Kondensator :   $f_{res4} = 0Hz$

Damit ergeben sich folgende Aussagen auf der Basis der ermittelten Eigenfrequenz der Schwingkreis-Anordnung:

- Ein Stent, bei dem kein Kondensator defekt ist, antwortet bei 673 Mhz.
- Ein Stent mit einem defekten und drei intakten Kondensatoren antwortet bei 581 Mhz.
- Ein Stent mit zwei defekten und zwei intakten Kondensatoren antwortet bei 475 Mhz.
- Ein Stent mit drei defekten Kondensatoren und einem intakten antwortet bei 336 Mhz.
- Ein Stent mit vier defekten Kondensatoren lässt keine Detektion der Eigenfrequenz zu.

Die vorstehenden Situationen und die entsprechenden Eigenfrequenzen sind in den Fig. 2a bis 2e sowie Fig. 2f dargestellt.

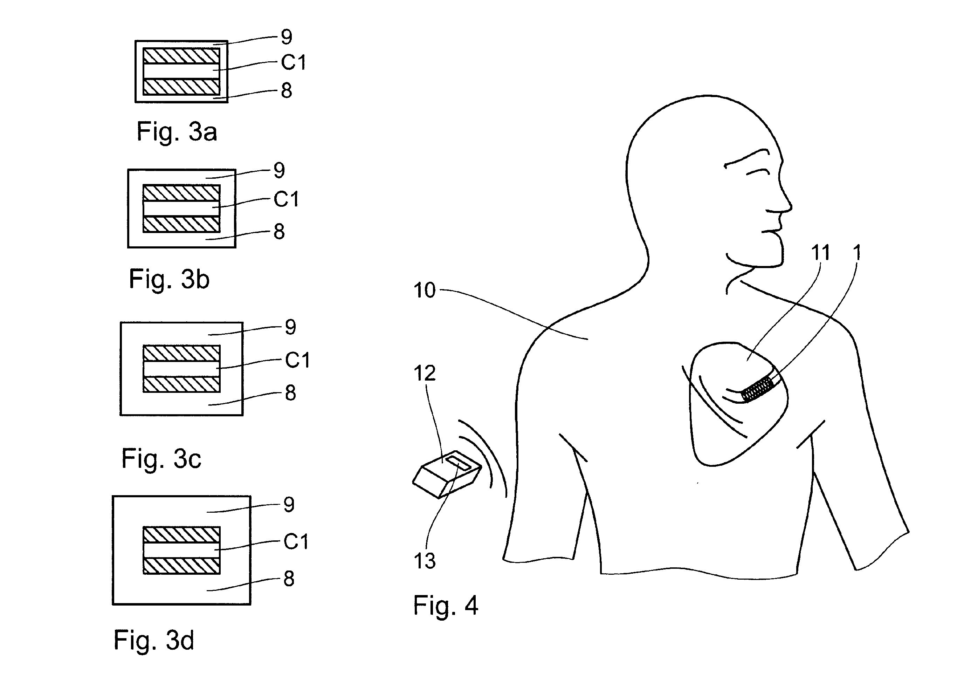

Wie aus Fig. 3a bis 3d deutlich wird, können die die Kondensatoren C1 bis C4 bildenden Streben 2 des Stents 1 mit unterschiedlich dicken Isolationslagen 8, 9 versehen sein. Bei gleichen bio-degradierbaren Materialien werden die dünnen Isolationslagen 8, 9 beim Ausführungsbeispiel gemäß Fig. 3a deutlich früher degradiert sein, als die zunehmend dickeren Isolationslagen 8, 9 bei den anderen Ausführungsbeispielen gemäß Fig. 3b bis 3d. Insoweit wird der entsprechende Kondensator deutlich früher kurzgeschlossen und damit aus der Schwingkreis-Anordnung 4 ausgeschaltet.

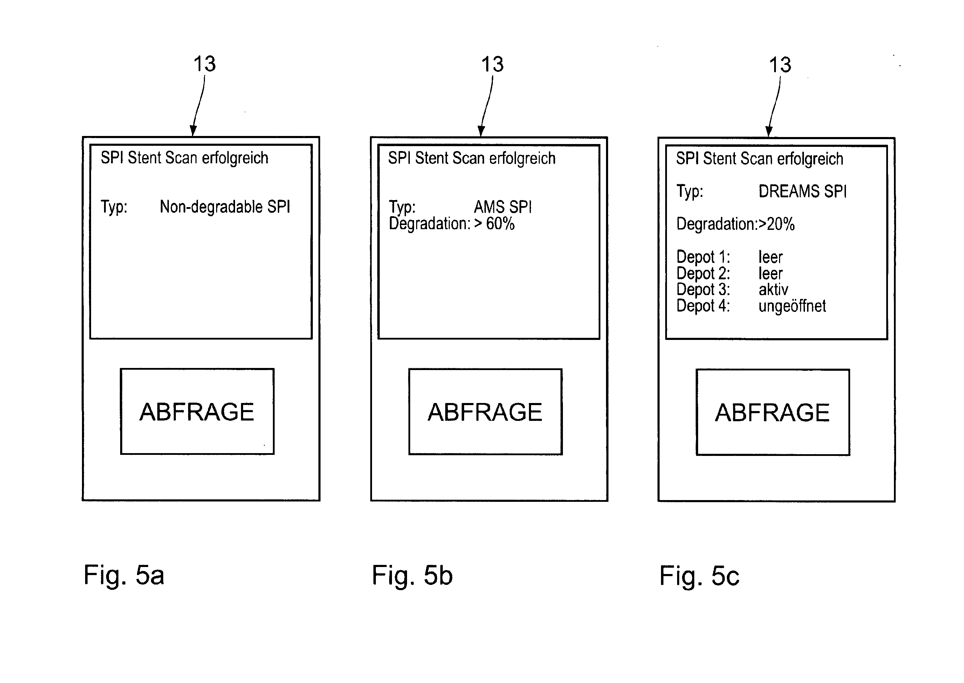

In Fig. 4 ist ein Patient 10 dargestellt, in dessen Herz 11 ein Stent 1 mit Schwingkreis-Anordnung 4 implantiert ist. Mit einem externen Abfragegerät 12 kann die Eigenfrequenz der Schwingkreis-Anordnung 4 im Stent 1 abgefragt werden. Dazu ist in dem Abfragegerät 12 ein Frequenz-Sweep-Generator vorgesehen, der eine elektromagnetische Strahlung mit einem Frequenzbereich von beispielsweise 200 bis 800 MHz aussendet. Je nach Zustand der einzelnen Kondensatoren C1 bis C4 in der Schwingkreis-Anordnung 4 wird der Schwingkreis mit einer der oben rechnerisch dargestellten Eigenfrequenzen "antworten", die vom Abfragegerät 12 detektierbar und in eine entsprechende Anzeige umsetzbar ist. Dazu ist auf dem Abfragegerät 12 ein Display 13 vorgesehen, das mit verschiedenen Meldungen in den Fig. 5a bis 5c dargestellt ist.

Handelt es sich bei dem Stent 1 um einen nicht-degradierbaren Stent, so kann dies durch eine Eigenfrequenz der Schwingkreis-Anordnung 4 signalisiert werden. Wird diese Eigenfrequenz vom Abfragegerät 12 ermittelt, erscheint auf dem Display 13 die in Fig. 5a wiedergegebene Typanzeige "Non degradable SPI", also "Nicht degradierbarer Stent".

Ist der Stent 1 vom Typ eines bio-degradierbaren Stents, so kann dies wiederum über die gemessenen Eigenfrequenzen erfasst und - wie in Fig. 5b gezeigt ist - entsprechend zur Anzeige in Form einer Typ- und Degradationsgrad-Information gebracht werden.

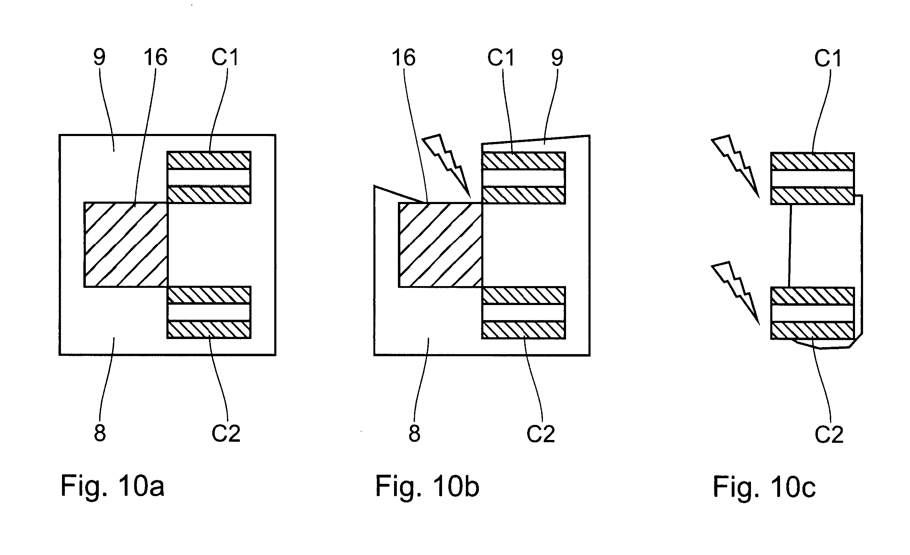

Fig. 5c zeigt die Anzeige im Display 13 bei einem Stent 1, in den vier Medikamentendepots integriert sind. Wie später noch anhand von Fig. 10 näher erläutert wird, kann über entsprechende Kondensatoren C1 bis C4 der Zustand des jeweiligen Depots detektiert und vom Abfragegerät 12 auf der Basis der gemessenen Eigenfrequenz abhängig von der Kurzschlusssituation der den Medikamentendepots zugeordneten Kondensatoren ermittelt werden.

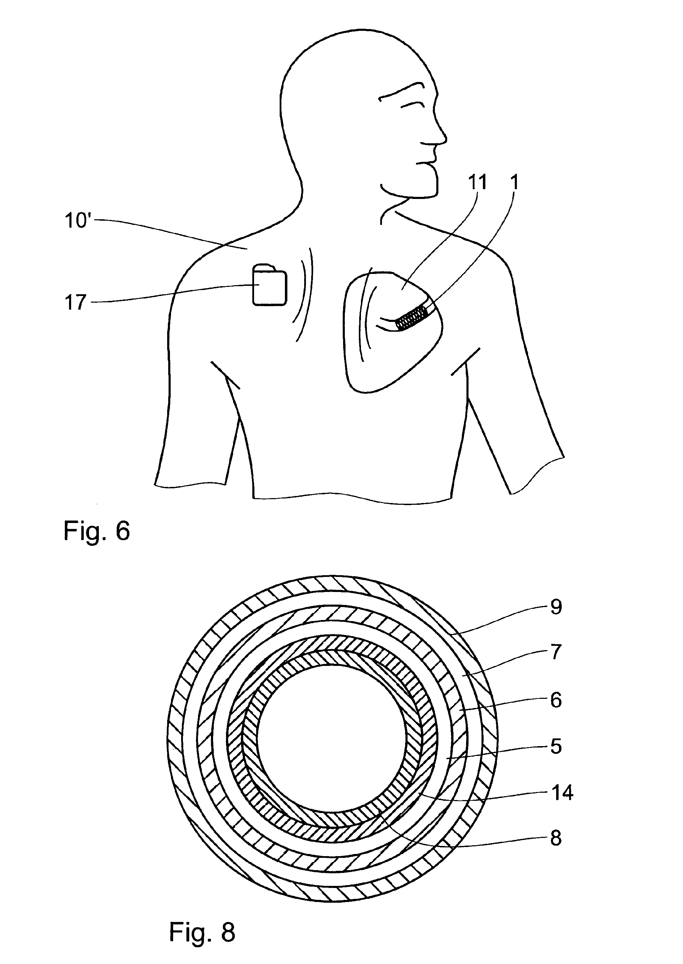

Alternativ zu dem Abfragegerät 12 kann die Abfrage auch durch ein aktives medizinisches Implantat, wie einen Herzschrittmacher 17, absolviert werden, wie dies in Fig. 6 angedeutet ist. Dieser sendet dann die ermittelten Informationen telemetrisch an ein externes, nicht näher dargestelltes Basisgerät.

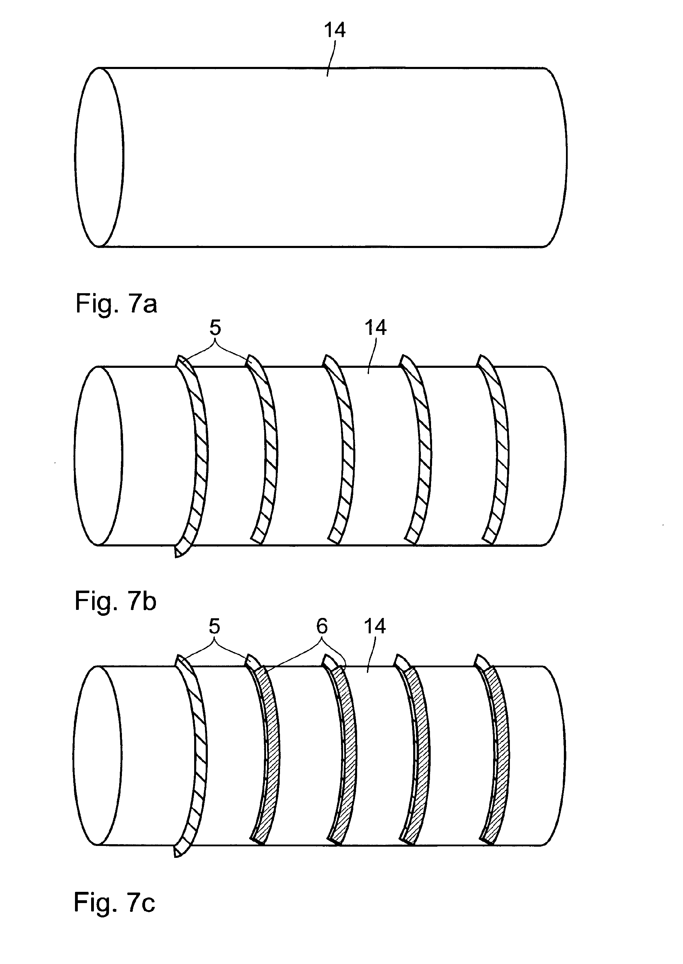

Anhand der Fig. 7a bis 7f und Fig. 8 ist nun stark schematisiert ein möglicher Herstellungsweg für den Stent 1 mit integrierter Schwingkreis-Anordnung 4 und dessen Lagenaufbau erläuterbar. Die Abbildungen sind dabei stark vereinfacht, indem beispielsweise die üblicherweise bei Stents vorhandenen Dehnungselemente nicht mit dargestellt sind.

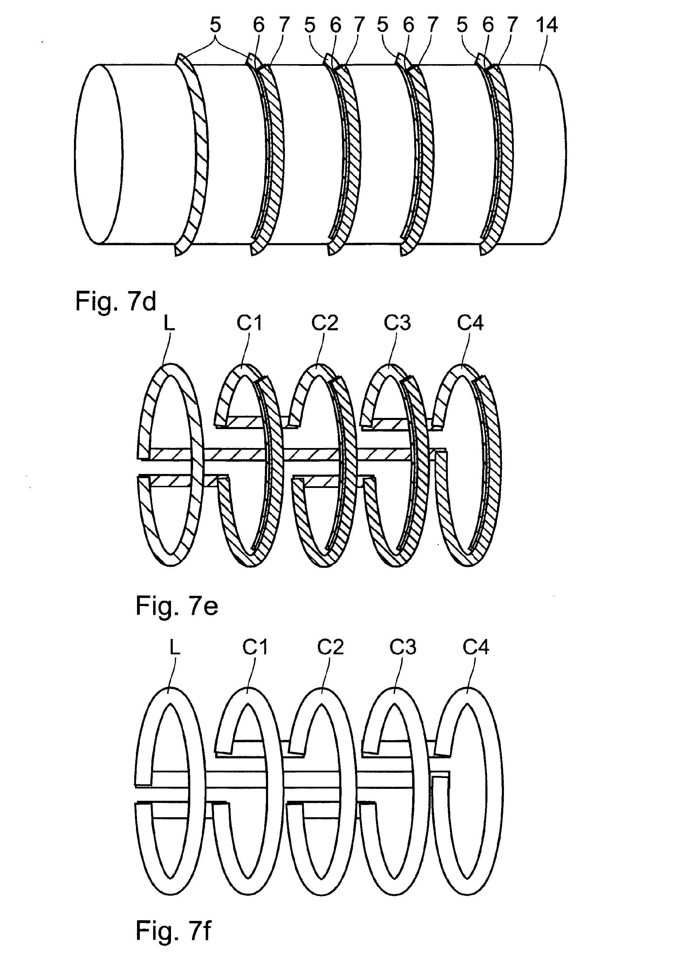

Wie aus Fig. 7a hervorgeht, wird von einem röhrenförmigen Grundkörper 14 ausgegangen, der die nachfolgenden Lagen des Stents 1 aufnimmt und im fertigen Produkt als Basisstruktur der jeweiligen Strebe fungiert. Auf diesen Grundkörper 14 werden in Umfangsrichtung U laufende, teilringförmige elektrisch leitfähige Lagen 5 als Leiterbahnen aufgebracht, die die Spule L und einen Kondensatorpol der Kondensatoren C1 bis C4 bilden (Fig. 7b). Auf eine Teilumfangslänge dieser inneren elektrisch leitenden Lage 5 wird bei den Kondensatoren C1 bis C4 eine Zwischenlage 6 aus einem dielektrischen Material aufgebracht (Fig. 7c). Danach wird bei den Kondensatoren C1 bis C4 wiederum überlappend mit dieser Zwischenlage 6 jeweils eine teilringförmige äußere elektrisch leitfähige Lage 7 aufgebracht (Fig. 7d).

Wie aus Fig. 7e deutlich wird, wird aus diesem Rohling die eigentliche Stentstruktur ausgeschnitten und anschließend allseitig mit einer Isolierung in Form einer inneren und äußeren Isolationslage 8, 9 umgeben (siehe Fig. 7f). Insgesamt ergibt sich der in Fig. 8 äußerst schematisch dargestellte Schichtaufbau im Querschnitt. Die entsprechenden Lagen sind von innen nach außen entsprechend dem oben erörterten Herstellungsverfahren die innere Isolierlage 8, der Grundkörper 14, die innere, elektrisch leitfähige Lage 5 als ein Kondensatorpol, die dielektrische Zwischenlage 6, die äußere, elektrisch leitfähige Lage 7 als zweiter Kondensatorpol und die äußere Isolierlage 9.

Falls die Isolierlagen 8, 9, der Grundkörper 14 und die Zwischenlage 6 aus einem einheitlichen Werkstoff einerseits und die beiden elektrisch leitfähigen Lagen 5, 7 aus einem elektrischen Leiter andererseits bestehen, können im günstigsten Fall für den Stent nur zwei Werkstoffe zum Einsatz kommen, die bei Stent-Konstruktionen grundsätzlich bekannt sind.

Für die Grundkörper 14 und Isolierlagen 8, 9 können degradierbare, elektrisch nicht-leitende, biokompatible Materialien verwendet werden. Für die elektrisch leitfähigen Lagen 5, 7 sowie die Leiterschleife für die Induktivität L sind vorzugsweise biokompatible, elektrisch leitende Werkstoffe zu verwenden, wobei es sich beispielsweise um bereits fur Stents verwendete Metalle handeln kann, wie Eisen, Magnesium oder deren Legierungen. Auch elektrisch leitende Polymere sind für die Lagen 5, 7 denkbar.

Für die Zwischenlage 6 können ebenfalls degradierbare, elektrisch nicht-leitende biokompatible Materialien verwendet werden.

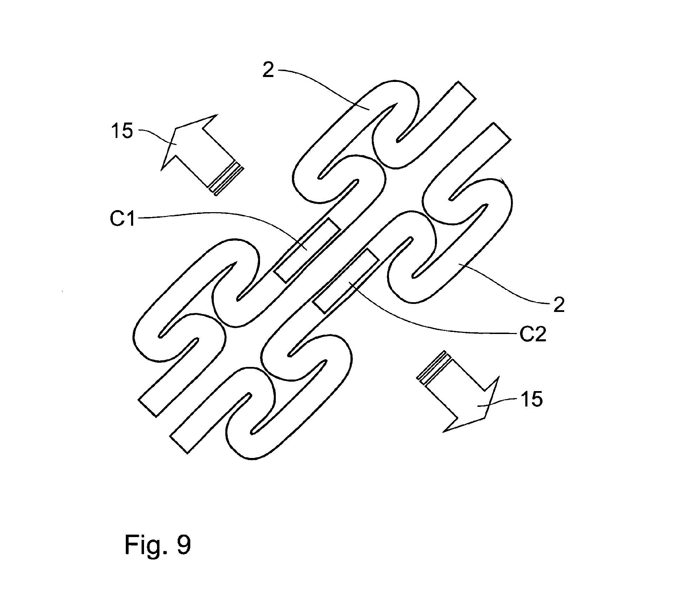

Wie aus Fig. 9 deutlich wird, können die Kondensatoren C1, C2 der Schwingkreis-Anordnung 4 vorzugsweise in mechanisch wenig belasteten Positionen des Stents 1 untergebracht werden, also in Zonen, die bei der Dilatation (siehe Pfeile 15 in Fig. 9) nicht deformiert werden.

Anhand von Fig. 10a bis 10c wird nun die Funktionsweise der Schwingkreis-Anordnung 4 im Zusammenhang mit der Detektion der Aktivität eines Medikamentendepots in einem Stent näher erläutert. Fig. 10a zeigt einen Querschnitt durch eine Strebe 2 eines Stents 1 am Ort eines Medikamentendepots 16, das gemeinsam mit zwei flankierenden Kondensatoren C1, C2 in eine bio-degradierbare Isolation 8, 9 eingebettet ist. Im Ausgangszustand gemäß Fig. 10a ist keiner der beiden Kondensatoren C1, C2 kurzgeschlossen und trägt damit zum Schwingkreis bei. Es stellt sich eine bestimmte Eigenfrequenz des Schwingkreises ein.

In Fig. 10b hat sich die Isolierlage 9 außen bereits so abgebaut, dass das Medikamentendepot 16 freiliegt und somit eine Wirkstoffabgabe stattfindet. Der im Bereich der abgebauten Isolierlage 9 sitzende Kondensator C1 liegt damit ebenfalls frei und wird durch die Körperflüssigkeit kurzgeschlossen. Damit ändert sich die Eigenfrequenz des Schwingkreises signifikant, was durch ein Abfragegerät 12 ermittelt werden kann. Der entsprechende Sprung in der Eigenfrequenz weist darauf hin, dass die Medikamentenabgabe aus dem Depot 16 aktiv ist.

Bei vollständigem Abbau des Medikamentendepots 16 und entsprechendem Zerfall der Isolierlagen 8, 9 wird nach einer gewissen Zeit auch der zweite Kondensator C2 freigelegt und damit kurzgeschlossen, was wiederum einen signifikanten Sprung in der Eigenfrequenz des Schwingkreises 4 mit sich bringt. Dies wird vom Abfragegerät dahingehend analysiert, dass nun die Medikamentenabgabe aus dem Depot 16 vollständig abgelaufen und das Depot damit leer ist.

Abschließend wird darauf hingewiesen, dass eine Auslegung der Kondensatoren C1 bis C4 und die Festlegung der Eigenfrequenz des daraus gebildeten Schwingkreises durch eine Änderung der Stegbreite oder -länge der die Kondensatorpole bildenden lagenförmigen Leiterbahnen, durch den Einsatz unterschiedlicher Dielektrika auf einem Stent oder auch eine Änderung der Dielektrikumsdicke vonstatten gehen kann. Letzteres ist beispielsweise durch eine exzentrische Auftragung des Dielektrikums realisierbar.

Auch sind unterschiedliche Serien- und Parallelschaltungen von Kondensatoren, Spulen oder auch Widerständen denkbar, um Schwingkreis-Anordnungen 4 mit spezifischen Eigenfrequenzen und Änderungsverhalten bei einer Freilegung von Elementen zu schaffen.

**Patentansprüche**

1. Medizinisches Implantat, insbesondere Stent zum Einsatz in Körperlumen, mit einer Basisstruktur (2, 3), wobei in die Basisstruktur (2, 3) eine passive elektronische Schwingkreis-Anordnung (4) aus mindestens einer Induktivität (L) und mindestens einem Kondensator (C1 bis C4) integriert ist, deren Eigenfrequenz ($f_{res}$) im implantierten Zustand des Implantats (1) von außen durch Anregung der Schwingkreis-Anordnung (4) mittels einer elektromagnetischen Strahlung derart erfassbar ist, dass eine Veränderung der Basisstruktur (2, 3) durch den Wert oder eine Änderung der Eigenfrequenz ($f_{res}$) der Schwingkreis-Anordnung (4) detektierbar sind, **dadurch gekennzeichnet, dass** die Eigenfrequenz ($f_{res}$) änderbar ist durch einen Kurzschluss des mindestens einen Kondensators (C1 bis C4), hervorgerufen durch die Biodegradierung einer die Schwingkreis-Anordnung (4) abdeckenden Isolation (8, 9) und Freilegung des Kondensators (C1 bis C4) gegenüber der im Körperlumen fließenden, physiologischen, elektrisch leitfähigen Flüssigkeit.

2. Implantat nach Anspruch 1, **dadurch gekennzeichnet, dass** die Eigenfrequenz ($f_{res}$) der Schwingkreis-Anordnung

(4) signifikant für den Typ oder die Seriennummer des Implantats (1) ist.

3. Implantat nach Anspruch 1, **dadurch gekennzeichnet, dass** eine Änderung der Eigenfrequenz ($f_{res}$) der Schwingkreis-Anordnung (4) nach der Implantation des Implantats (1) signifikant ist für eine Beschädigung der Basisstruktur (2, 3).

4. Implantat nach Anspruch 1, insbesondere biologisch abbaubares Implantat, **dadurch gekennzeichnet, dass** eine Änderung der Eigenfrequenz ($f_{res}$) der Schwingkreis-Anordnung (4) nach der Implantation des Implantats (1) signifikant ist für das Maß der Biodegradierung der Basisstruktur (2, 3).

5. Implantat nach Anspruch 1, insbesondere Implantat mit einem sich zeitlich verzögert abbauenden Medikamentendepot, **dadurch gekennzeichnet, dass** eine Änderung der Eigenfrequenz ($f_{res}$) der Schwingkreis-Anordnung (4) nach der Implantation des Implantats (1) signifikant ist für den Status eines in das Implantat (1) integrierten Medikamentendepots (16).

6. Implantat nach einem der vorgenannten Ansprüche, **dadurch gekennzeichnet, dass** die Schwingkreis-Anordnung eine Induktivität (L) in Kombination mit mehreren Kondensatoren (C1 bis C4), gegebenenfalls voneinander abweichender Kapazitätswerte, aufweist.

7. Implantat nach einem der vorgenannten Ansprüche, **dadurch gekennzeichnet, dass** die Induktivität (L) der Schwingkreis-Anordnung (4) durch eine elektrisch leitfähige Strebe (2, 3) der Basisstruktur (2, 3) gebildet ist.

8. Implantat nach einem der vorgenannten Ansprüche, **dadurch gekennzeichnet, dass** der mindestens eine Kondensator (C1 bis C4) der Schwingkreis-Anordnung (4) durch einen Lagenaufbau einer Strebe (2, 3) der Basisstruktur (2, 3) bestehend aus einer inneren Isolationslage (8), einer ersten elektrisch leitfähigen Lage (5) als Kondensatorpol, einer vorzugsweise als Dielektrikum ausgebildeten Zwischenlage (6), einer zweiten elektrisch leitfähigen Lage (7) als Kondensatorpol und einer äußeren, gegebenenfalls aus biodegradierbarem Material bestehenden Isolationslage (9) gebildet ist.

9. Implantat nach einem der vorgenannten Ansprüche, **dadurch gekennzeichnet, dass** die Eigenfrequenz ($f_{res}$) der Schwingkreis-Anordnung (4) von einem externen Abfragegerät (12) oder einem in den Körper implantierten aktiven medizinischen Implantat (17) detektierbar ist.

10. Implantat nach einem der vorgenannten Ansprüche, **dadurch gekennzeichnet, dass** die Frequenz der elektromagnetischen Strahlung zur Anregung der Schwingkreis-Anordnung in der Größenordnung von einigen Kilohertz bis Gigahertz, vorzugsweise zwischen 300 Mhz und 700 Mhz liegt.

**Claims**

1. A medical implant, in particular a stent for use in a bodily lumen, comprising a base structure (2, 3), wherein a passive electronic oscillating circuit arrangement (4) formed of at least one inductor (L) and at least one capacitor (C1 to C4) is integrated in the base structure (2, 3), the natural frequency ($f_{res}$) of said circuit arrangement in the implanted state of the implant (1) being externally determinable by excitation of the oscillating circuit arrangement (4) by means of an electromagnetic radiation such that a change of the base structure (2, 3) is detectable by the value or a change of the natural frequency ($f_{res}$) of the oscillating circuit arrangement (4), **characterised in that** the natural frequency ($f_{res}$) can be changed by a short circuit of the at least one capacitor (C1 to C4), caused by the biodegradation of an insulation (8, 9) covering the oscillating circuit arrangement (4) and exposure of the capacitor (C1 to C4) to the physiological, electrically conductive liquid flowing in the bodily lumen.

2. The implant according to Claim 1, **characterised in that** the natural frequency ($f_{res}$) of the oscillating circuit arrangement (4) is significant for the type or the serial number of the implant (1).

3. The implant according to Claim 1, **characterised in that** a change of the natural frequency ($f_{res}$) of the oscillating circuit arrangement (4) after the implantation of the implant (1) is significant for damage to the base structure (2, 3).

4. The implant according to Claim 1, in particular a biologically degradable implant, **characterised in that** a change of the natural frequency ($f_{res}$) of the oscillating circuit arrangement (4) after the implantation of the implant (1) is significant for the amount of biodegradation of the base structure (2, 3).

5. The implant according to Claim 1, in particular an implant having a drug reservoir degrading in a time-delayed manner, **characterised in that** a change of the natural frequency ($f_{res}$) of the oscillating circuit arrangement (4) after the implantation of the implant (1) is significant for the status of a drug reservoir (16) integrated in the implant (1).

6. The implant according to one of the preceding claims, **characterised in that** the oscillating circuit arrangement has an inductor (L) in combination with a plurality of capacitors (C1 to C4), possibly with capacitance values deviating from one another.

7. The implant according to one of the preceding claims, **characterised in that** the inductor (L) of the oscillating circuit arrangement (4) is formed by an electrically conductive strut (2, 3) of the base structure (2, 3).

8. The implant according to one of the preceding claims, **characterised in that** the at least one capacitor (C1 to C4) of the oscillating circuit arrangement (4) is formed by a layered construction of a strut (2, 3) of the base structure (2, 3) consisting of an internal insulation layer (8), a first electrically conductive layer (5) as a capacitor pole, an intermediate layer (6), preferably formed as a dielectric material, a second electrically conductive layer (7) as a capacitor pole, and an external insulation layer (9) possibly consisting of biodegradable material.

9. The implant according to one of the preceding claims, **characterised in that** the natural frequency ($f_{res}$) of the oscillating circuit arrangement (4) is detectable by an external scanning device (12) or an active medical implant (17) implanted in the body.

10. The implant according to one of the preceding claims, **characterised in that** the frequency of the electromagnetic radiation for exciting the oscillating circuit arrangement is in the order of magnitude of a few kilohertz to gigahertz, preferably between 300 MHz and 700 MHz.

**Revendications**

1. Implant médical, notamment stent, pour l'insertion dans des lumens corporels, avec une structure de base (2, 3), où un ensemble circuit oscillant (4) passif électronique constitué d'au moins une inductance (L) et d'au moins un condensateur (C1 à C4) est intégré dans la structure de base (2, 3), dont la fréquence propre ($f_{res}$) de l'implant (1) à l'état implanté peut être évaluée de l'extérieur par une sollicitation de l'ensemble circuit oscillant (4) au moyen d'un rayonnement électromagnétique de telle manière qu'une modification de la structure de base (2, 3) peut être détectée par la valeur ou une modification de la fréquence propre ($f_{res}$) de l'ensemble circuit oscillant (4), **caractérisé en ce que** la fréquence propre ($f_{res}$) peut être modifiée par un court-circuit de l'au moins un condensateur (C1 à C4), engendré par la biodégradation d'une isolation (8, 9) recouvrant l'ensemble circuit oscillant (4) et la libération du condensateur (C1 à C4) par rapport au liquide physiologique électriquement conducteur circulant dans le lumen corporel.

2. Implant selon la revendication 1, **caractérisé en ce que** la fréquence propre ($f_{res}$) de l'ensemble circuit oscillant (4) est significative pour le type ou le numéro de série de l'implant (1).

3. Implant selon la revendication 1, **caractérisé en ce qu'**une modification de la fréquence propre ($f_{res}$) de l'ensemble circuit oscillant (4) après l'implantation de l'implant (1) est significative pour un endommagement de la structure de base (2, 3).

4. Implant selon la revendication 1, notamment implant biologiquement dégradable, **caractérisé en ce qu'**une modification de la fréquence propre ($f_{res}$) de l'ensemble circuit oscillant (4) après l'implantation de l'implant (1) est significative pour la mesure de la biodégradation de la structure de base (2, 3).

5. Implant selon la revendication 1, notamment implant avec un dépôt de médicament se dégradant de manière différée dans le temps, **caractérisé en ce qu'**une modification de la fréquence propre ($f_{res}$) de l'ensemble circuit oscillant (4) après l'implantation de l'implant (1) est significative pour un état du dépôt de médicament (16) intégré dans l'implant (1).

6. Implant selon l'une des revendications précitées, **caractérisé en ce que** l'ensemble circuit oscillant (4) présente une inductance (L) en combinaison avec plusieurs condensateurs (C1 à C4) de valeurs de capacités éventuellement différentes les unes des autres.

7. Implant selon l'une des revendications précitées, **caractérisé en ce que** l'inductance (L) de l'ensemble circuit oscillant (4) est formée par une entretoise (2, 3) électriquement conductrice de la structure de base (2, 3).

8. Implant selon l'une des revendications précitées, **caractérisé en ce que** l'au moins un condensateur (C1 à C4) de l'ensemble circuit oscillant (4) est formé par une construction en longueur d'une entretoise (2, 3) de la structure de base (2, 3) constituée d'une couche d'isolation (8) interne, d'une première couche électriquement conductrice (5) en tant que pôle de condensateur, d'une couche intermédiaire (6) conçue de préférence sous forme de diélectrique, d'une deuxième couche électriquement conductrice (7) en tant que pôle de condensateur et d'une couche d'isolation (9) externe, éventuellement constituée d'un matériau biodégradable.

9. Implant selon l'une des revendications précitées, **caractérisé en ce que** la fréquence propre ($f_{res}$) de l'ensemble circuit oscillant (4) peut être détectée par un appareil interrogateur (12) externe ou un implant (17) médical actif implanté dans le corps.

10. Implant selon l'une des revendications précitées, **caractérisé en ce que** la fréquence du rayonnement électromagnétique pour la sollicitation de l'ensemble circuit oscillant se situe dans l'ordre de grandeur de quelques kilohertz à gigahertz, de préférence entre 300 MHz et 700 MHz.

Fig. 1a

Fig. 1b

Fig. 2a  Fig. 2b  Fig. 2c  Fig. 2d  Fig. 2e

fres0  fres1  fres2  fres3  fres4

Fig. 2f

EP 1 990 027 B1

Fig. 4

Fig. 3a

Fig. 3b

Fig. 3c

Fig. 3d

13

SPI Stent Scan erfolgreich

Typ:      Non-degradable SPI

ABFRAGE

Fig. 5a

13

SPI Stent Scan erfolgreich

Typ:          AMS SPI
Degradation: > 60%

ABFRAGE

Fig. 5b

13

SPI Stent Scan erfolgreich

Typ:          DREAMS SPI

Degradation:>20%

Depot 1:    leer
Depot 2:    leer
Depot 3:    aktiv
Depot 4:    ungeöffnet

ABFRAGE

Fig. 5c

EP 1 990 027 B1

Fig. 6

Fig. 8

14

Fig. 7a

5    14

Fig. 7b

5    6    14

Fig. 7c

Fig. 7d

Fig. 7e

Fig. 7f

Fig. 9

Fig. 10c

Fig. 10b

Fig. 10a